# EUROPÄISCHE PATENTSCHRIFT

(11) **EP 2 691 766 B1**
(45) Veröffentlichungstag und Bekanntmachung des Hinweises auf die Patenterteilung: **31.12.2014**
(21) Anmeldenummer: 12713084.7
(22) Anmeldetag: 29.03.2012
(51) Int. Cl.: G01N 30/28, G01N 30/86, G01N 30/32, G01N 30/34, G01N 30/66, G01N 33/18

(54) **VERFAHREN ZUR CHROMATOGRAPHISCHEN ANALYSE EINES WASSERSTOFF ENTHALTENDEN GASGEMISCHS**
METHOD FOR A CHROMATOGRAPHIC ANALYSIS OF A HYDROGEN-CONTAINING GAS MIXTURE
PROCÉDÉ POUR L'ANALYSE CHROMATOGRAPHIQUE D'UN MÉLANGE GAZEUX CONTENANT DE L'HYDROGÈNE

(30) Priorität: 30.03.2011 DE 102011006452
(43) Veröffentlichungstag der Anmeldung: 05.02.2014
(73) Patentinhaber: Siemens Aktiengesellschaft, 80333 München (DE)
(72) Erfinder: PROBST, Frank, 76863 Herxheim bei Landau/Pfalz (DE); STRAUCH, Piotr, 76761 Rülzheim (DE)
(86) Internationale Anmeldenummer: PCT/EP2012/055587
(87) Internationale Veröffentlichungsnummer: WO 2012/130923

(56) Entgegenhaltungen:
- DE-B- 1 223 176
- DE-B3-102008 061 158
- US-A- 3 585 002
- US-A- 4 464 925
- CASTELLO G ET AL: "The quantitative determination of hydrogen in gases, by gas chromatography with helium as the carrier gas", JOURNAL OF CHROMATOGRAPHY, ELSEVIER SCIENCE PUBLISHERS B.V, NL, Bd. 20, 1. Januar 1965 (1965-01-01), Seiten 447-451, XP026749577, ISSN: 0021-9673, DOI: 10.1016/S0021-9673(01)97446-1 [gefunden am 1965-01-01]

## Beschreibung

Die Erfindung betrifft ein Verfahren zur chromatographischen Analyse eines Wasserstoff enthaltenden Gasgemischs.

Bei der Gaschromatographie wird eine Dosis eines zu analysierenden Gasgemischs in einen Trägergasstrom eingebracht und mittels diesen durch eine aus einer oder mehreren Trennsäulen bestehende Trenneinrichtung geführt. Aufgrund unterschiedlicher Wechselwirkung mit dem Material der Trenneinrichtung treten die verschiedenen Komponenten des Gasgemischs voneinander getrennt nacheinander aus der Trenneinrichtung aus. Vorzugsweise wird am Ausgang der Trenneinrichtung ein Wärmeleitfähigkeitsdetektor zur Detektion der ankommenden getrennten Komponenten verwendet. Zum einen lassen sich mit einem Wärmeleitfähigkeitsdetektor prinzipiell alle Gaskomponenten nachweisen, was insbesondere bei einer Vielzahl von nachzuweisenden Komponenten von Vorteil ist; zum anderen benötigt ein Wärmeleitfähigkeitsdetektor außer dem Trägergas keine weiteren Betriebsgase. Der Nachweis der einzelnen Komponenten mit dem Wärmeleitfähigkeitsdetektor basiert auf ihren unterschiedlichen Wärmeleitfähigkeiten gegenüber der des Trägergases. Als Trägergas wird häufig Helium, aber auch Stickstoff, Argon oder Wasserstoff eingesetzt. Wasserstoff und Helium ermöglichen mit ihrem hohen Wärmeleitfähigkeitsunterschied zu anderen Komponenten einen besonders empfindlichen Nachweis dieser anderen Komponenten.

Die gaschromatographische Analyse von Brenngasen beinhaltet neben dem Nachweis einer Vielzahl unterschiedlicher Kohlenwasserstoffe sowie von (u. a.) Sauerstoff, Stickstoff und Kohlendioxid auch den Nachweis von Wasserstoff. Damit kommt praktisch nur Helium als Trägergas in Frage. Hierbei tritt das Problem auf, dass Helium als Trägergas keine eindeutige Detektion von Wasserstoff mittels Wärmeleitfähigkeitsdetektor ermöglicht.

Mit Ausnahme von Wasserstoff ist die Wärmeleitfähigkeit der nachzuweisenden Komponenten kleiner als die des Trägergases Helium, so dass der Wärmeleitfähigkeitsdetektor bei der Detektion jeder dieser Komponenten ein Signal in Form eines positiven Peaks erzeugt, dessen Fläche proportional zur Konzentration der detektierten Komponente ist. Da die Wärmeleitfähigkeit von Wasserstoff größer als die von Helium ist, wäre ein negativer Peak zu erwarten. Tatsächlich erhält man bei geringen Konzentrationen bis etwa 5 bis 10 % Wasserstoff in Helium einen positiven Peak. Danach bildet sich bei höheren Wasserstoffkonzentrationen an der Spitze des Peaks ein Tal aus, das mit weiter zunehmender Konzentration des Wasserstoffs zu einem immer größer werdenden negativen Peak auswächst. Wird also ein entsprechendes Wasserstoff-Helium-Gemisch als Trägergas verwendet, so erhält man bei jeder Konzentration der nachzuweisenden Komponente Wasserstoff einen negativen Peak. Dieser Lösungsansatz ist z. B. aus Don E. Clay et al.: "Single Detector Analysis of Refinery Gases", Application Note 10090, Thermo Electron Corporation, gefunden am 21. Januar 2011 im Internet unter http://www.thermo.com/eThermo/CMA/PDFs/Articles/articlesFile 25894.pdf, bekannt, wobei eine Trägergasmischung aus 8,5 % Wasserstoff in Helium verwendet wird und das negative Signal des Wärmeleitfähigkeitsdetektors bei Erscheinen der Wasserstoffkomponente invertiert wird. Ein solches Gasgemisch ist nicht marktüblich und seine Herstellung mit entsprechend hoher Genauigkeit ist kostenaufwendig.

Alternativ zur Lösung des oben genannten Problems kann mit unterschiedlichen Trägergasen für den Nachweis des Wasserstoffs und den der anderen Komponenten gearbeitet werden. So ist es möglich, nach einer doppelten Probennahme die eine Probe in einer Trenneinrichtung mit den Trägergas Helium auf Kohlenwasserstoffe zu untersuchen und die zweite Probe entweder anschließend in derselben Trenneinrichtung oder parallel in einer weiteren Trenneinrichtung mit dem Trägergas Argon oder Stickstoff auf Wasserstoff zu untersuchen.

Aus der JP 6-258306 A ist es bekannt, bei der gaschromatographischen Analyse eines Wasserstoff und Kohlenwasserstoffe enthaltenden Gasgemischs das verwendete Trägergas während der Analyse zu wechseln. Dabei wird zunächst die Probe mittels Stickstoff als erstem Trägergas durch eine aus zwei Trennabschnitten bestehende Trenneinrichtung geleitet, an deren Ende der Wasserstoff detektiert und quantitativ bestimmt wird. Anschließend werden die Kohlenwasserstoffe, nachdem sie in den zweiten Trennabschnitt gelangt sind, durch diesen mittels Helium als zweitem Trägergas zu dem Detektor geleitet, während der erste Trennabschnitt mit dem Stickstoff rückgespült wird. Die Trägergasumschaltung inmitten der Trenneinrichtung kann problematisch sein, weil die Kohlenwasserstoffe zunächst noch in dem Stickstoff enthalten sind und erst im weiteren Verlauf des zweiten Trennabschnitts von dem Helium erfasst werden.

Aus der DE 10 2008 061 158 B1 ist es bekannt, eine Probe eines Wasserstoff und Kohlenwasserstoffe enthaltenden Gasgemischs mittels Helium als erstem Trägergas in eine Trenneinrichtung zu leiten und, nachdem die Komponente Wasserstoff zumindest einen Teil der Trenneinrichtung durchlaufen hat, diesen Teil der Trenneinrichtung mit Argon oder Stickstoff als zweitem Trägergas zu spülen, und anschließend mittels des zweiten Trägergases eine weitere Probe in die Trenneinrichtung zu leiten. Alternativ wird Argon oder Stickstoff als erstes Trägergas verwendet und, nachdem die interessierenden Kohlenwasserstoffe zumindest einen Teil der Trenneinrichtung durchlaufen haben, dieser Teil der Trenneinrichtung mit Helium als zweitem Trägergas zu spülen, und anschließend mittels des zweiten Trägergases eine weitere Probe in die Trenneinrichtung geleitet.

CASTELLO G ET AL: "The quantitative determination of hydrogen in gases, by gas chromatography with helium as the carrier gas", J. Chrom., Bd. 20, 1. Januar 1965 (1965-01-01), S. 447-451 offenbart ein Kalibrationsverfahren mit anschließender Ermittlung des Wasserstoffs in einem Gasgemisch.

Der Erfindung liegt die Aufgabe zugrunde, bei der chromatographischen Analyse eines Gasgemischs den Wasserstoffgehalt eines Gasgemischs bis zu einer Konzentration von etwa 5 bis 6% zuverlässig und genau zu bestimmen, ohne dass dabei die Bestimmung weiterer Komponenten des Gasgemischs beeinträchtigt wird oder ein Trägergaswechsel erforderlich ist. Wasserstoffkonzentrationen über 5 bis 6% sollen ebenfalls, wenn auch mit hohem Messfehler, gemessen werden können.

Gemäß der Erfindung wird die Aufgabe durch das in Anspruch 1 angegebene Verfahren gelöst.

Vorteilhafte Weiterbildungen des erfindungsgemäßen Verfahrens sind in den Unteransprüchen angegeben.

Gegenstand der Erfindung ist somit ein Verfahren zur chromatographischen Analyse eines Wasserstoff enthaltenden Gasgemischs, wobei
- in einem ersten Messdurchgang eine erste Dosis des Gasgemischs mittels Helium als Trägergas durch eine Trenneinrichtung geleitet wird und die Konzentrationen interessierender getrennter Komponenten gemessen werden, wobei mittels eines Wärmeleitfähigkeitsdetektors die Konzentration des Wasserstoffs in dem Gasgemisch gemessen und der dabei erhaltene Messwert bis zu einem oberen Grenzwert im Bereich von 5 % bis 6 % übernommen wird,
- in einem zweiten Messdurchgang eine zweite Dosis desselben Gasgemischs mittels desselben Trägergases durch die Trenneinrichtung geleitet wird, wobei im Unterschied zu dem ersten Messdurchgang die Dosis verringert, das Gasgemisch volumetrisch verdünnt und/oder die Trägergasgeschwindigkeit verringert ist, und erneut mittels des Wärmeleitfähigkeitsdetektors die Konzentration des Wasserstoffs in dem Gasgemisch gemessen wird, und
- der aus dem ersten Messdurchgang übernommene Messwert mit dem in dem zweiten Messdurchgang erhaltenen Messwert verifiziert wird.

In dem ersten Messdurchgang können alle interessierenden Komponenten des zu analysierenden Gasgemischs einschließlich des Wasserstoffs mit hoher Genauigkeit gemessen werden. Der bis etwa 5 bis 6 % reichende Messbereich für Wasserstoff ist in der Praxis oft ausreichend. In dem zweiten Messdurchgang wird im Prinzip nur noch Wasserstoff gemessen. Es ist denkbar, auch noch beispielsweise Methan mit zu bestimmen und als Referenz für Normierungszwecken zu verrechnen. Aufgrund der geringeren Dosierung, volumetrischen Verdünnung des Gasgemischs und/oder verringerten Trägergasgeschwindigkeit wird zwar der Messfehler für Wasserstoffkonzentrationen unterhalb von etwa 10 % erhöht, aber der Messbereich erweitert. Damit kann anhand des im zweiten Messdurchgang erhaltenen Messwertes überprüft werden, ob in dem ersten Messdurchgang eine Überschreitung des Messbereichs stattgefunden hat, diese aber aufgrund der Zweideutigkeit des ersten Messwertes nicht erkannt worden ist.

Bei Wasserstoffkonzentrationen oberhalb von etwa 10 % wird der Messfehler des zweiten Messdurchgangs größer als im ersten Messdurchgang. Damit können in dem zweiten Messdurchgang Wasserstoffkonzentrationen über dem Grenzwert im Bereich von 5 % bis 6 % des ersten Messdurchgangs hinaus bis z. B. 20 % gemessen werden.

Zur weiteren Erläuterung der Erfindung wird im Folgenden auf die Figuren der Zeichnung Bezug genommen; Im Einzelnen zeigen:
- Figur 1: ein einfaches Beispiel eines Gaschromatographen zur Durchführung des erfindungsgemäßen Verfahrens,
- Figur 2: ein Beispiel für das Signal eines Wärmeleitfähigkeitsdetektors bei Detektion einer Wasserstoff-Fraktion in Helium,
- Figur 3: in einem Diagramm beispielhaft den Zusammenhang zwischen der Konzentration des Wasserstoffs in einem zu analysierenden Gasgemisch und dem durch Wärmeleitfähigkeitsmessung der chromatographisch abgetrennten Wasserstoff-Fraktion erhaltenen Messwert und
- Figur 4: einen vergrößerten Ausschnitt aus dem Diagramm nach Figur 3.

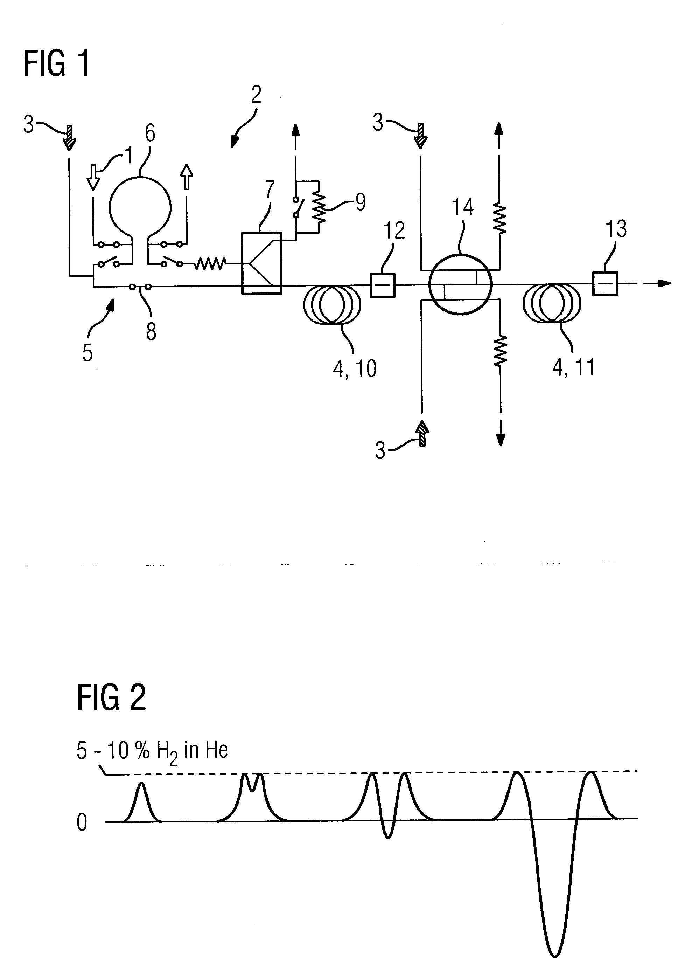

Bei dem in Figur 1 in schematischer Darstellung gezeigten Gaschromatograph wird ein zu analysierendes Gasgemisch 1 nach Entnahme aus einem technischen Prozess einer Dosiereinrichtung 2 zugeführt. Die Dosiereinrichtung 2 dient dazu, zu einem vorgegebenen Zeitpunkt eine vorgegebene Dosis des Gasgemischs 1 in Form eines kurzen und scharf begrenzten Probenpfropfes in einen Trägergasstrom 3 einzuschleusen und einer Trenneinrichtung 4 zuzuführen. Die Dosiereinrichtung 2 weist ein Dosierventil 5 auf, welches in einer hier gezeigten ersten Schaltstellung das Gasgemisch 1 in eine Probenschleife 6 leitet. In einer zweiten Schaltstellung wird die Probenschleife 6 in den Weg für das Trägergas 3 geschaltet, das die in der Probenschleife 6 enthaltene Probe des Gasgemischs 1 einem Injektor 7 zuführt. Solange ein Magnetventil 8 geöffnet ist, fließt das Trägergas 3 durch das Magnetventil 8 und den Injektor 7 in die Trenneinrichtung 4, während die Probe aus der Probenschleife 6 über eine Strömungsdrossel 9 nach außen abgeleitet wird. Wird das Magnetventil 8 für eine vorgegebene Dauer geschlossen, so wird in dem Injektor 7 aus der Probe ein Teil abgezweigt und als scharf begrenzter Probenpfropf in den Trägergasstrom 3 zur Trenneinrichtung 4 eingeschleust. Beim Durchströmen der Trenneinrichtung 4 werden die in dem Probenpfropf enthaltenen Komponenten des Gasgemischs 1 getrennt.

Die hier beispielhaft gezeigte Trenneinrichtung 4 besteht aus zwei oder mehr aufeinander folgenden Trennanordnungen 10, 11, die ihrerseits jeweils eine oder mehrere hintereinander geschaltete Trennsäulen mit unterschiedlichen Trenneigenschaften aufweisen. Am Ende jeder Trennanordnung 10, 11 bzw. jeder Trennsäule ist jeweils ein Wärmeleitfähigkeitsdetektor 12, 13 zur Detektion von bis dahin vollständig getrennten interessierenden Komponenten des Gasgemischs 1 angeordnet. Zwischen den Trennanordnungen 10 und 11 ist eine Gasumschalteinrichtung 14 eingefügt, die es ermöglicht, bis dahin vollständig getrennte und detektierte Komponenten aus der Trenneinrichtung 4 auszuschleusen und beide Trennanordnungen 10 und 11 voneinander unabhängig mit dem Trägergas 3 zu spülen.

Das Gasgemisch 1 handelt es sich beispielsweise um Brenngas, in dem mehrere Komponenten, darunter auch Wasserstoff, quantitativ nachgewiesen werden sollen. Als Trägergas 3 dient reines Helium. Zur Trennung des Wasserstoffs von z. B. Stickstoff und Methan ist die hintere Trennanordnung 11 als Molekularsieb ausgebildet. Die Detektion von Wasserstoff, Stickstoff und Methan erfolgt somit in dem Wärmeleitfähigkeitsdetektor 13. Stickstoff und Methan haben jeweils geringere Wärmeleitfähigkeiten als Helium, so dass der Wärmeleitfähigkeitsdetektor 13 bei Detektion jedes Mal ein positives Peaksignal erzeugt, dessen Fläche (oder auch Höhe) proportional zu der Stickstoff- bzw. Methanmenge ist. Demgegenüber verhält sich die Wärmeleitfähigkeit eines Wasserstoff-Helium-Gemischs völlig verschieden und anormal.

Figur 2 zeigt vier Peaksignale eines Wärmeleitfähigkeitsdetektors bei von links nach rechts zunehmender Konzentration von Wasserstoff in Helium. Bis zu einer Konzentration von etwa 5 bis 10 % steigt die Wärmeleitfähigkeit des Gemischs an, um danach mit weiter steigender Konzentration von Wasserstoff in Helium abzufallen. Als Folge bildet sich bei höheren Wasserstoffkonzentrationen an der Spitze des Peaksignals ein Tal aus, das mit weiter zunehmender Konzentration des Wasserstoffs zu einem immer größer werdenden negativen Peak auswächst. Die Fläche unter den vier Peaks nimmt also anfänglich zu, um danach abzufallen, so dass bei der Wasserstoffmessung Zweideutigkeiten auftreten.

Entsprechend dem erfindungsgemäßen Verfahren wird nun in einem ersten Messdurchgang eine erste Dosis des Gasgemischs 1 mittels des Trägergases Helium 3 durch die Trenneinrichtung 4 geleitet, wobei die Konzentrationen der interessierenden Komponenten gemessen werden. Die Dosis, d. h. der injizierte Probenpfropf, und die Trägergasgeschwindigkeit sind im Hinblick auf eine schnelle und optimale Trennung und Detektion der Komponenten bemessen. Die Molekularsiebsäule 11 ist mit 6 m gerade ausreichend lang, um den Wasserstoff von den anderen Komponenten ausreichend abzutrennen, so dass die abgetrennte Wasserstoff-Fraktion die Form eines relativ scharfen Peaks hat. Die Spitzenkonzentration des Wasserstoffs in dem Helium liegt daher im Größenbereich der Konzentration des Wasserstoffs in dem zu analysierenden Gasgemisch 1.

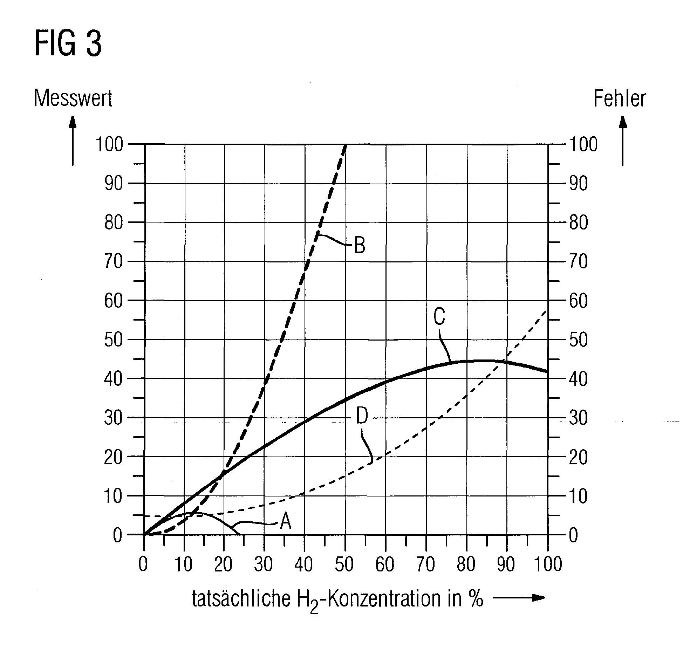

In Figur 3 zeigt die Kurve A beispielhaft den Zusammenhang zwischen der tatsächlichen Konzentration des Wasserstoffs in dem Gasgemisch und dem durch die Wärmeleitfähigkeitsmessung erhaltenen Messwert (Peakfläche). Bis zu einer Konzentration von etwa 5 bis 6 % ist dieser Zusammenhang linear. Darüber hinaus bis etwa 10 % erhält man eine weiterhin monoton steigende, aber nicht mehr lineare Funktion. Jenseits von etwa 10 % würde der Messwert trotz steigender Wasserstoffkonzentration wieder abnehmen und daher einen sehr hohen Messfehler annehmen. Die Kurve B zeigt den zur Kurve A gehörigen absoluten Messfehler.

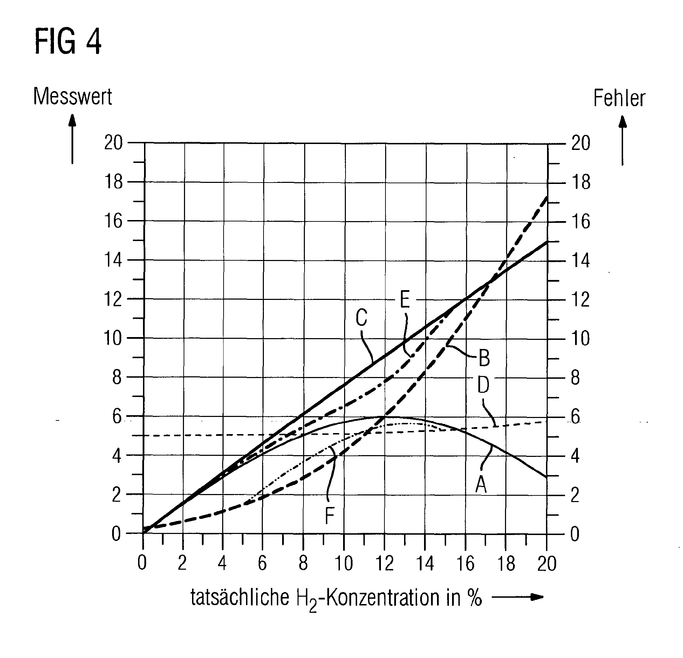

Figur 4 zeigt einen vergrößerten Ausschnitt des in Figur 3 gezeigten Diagramms für Konzentrationen des Wasserstoffs in dem Gasgemisch 1 bis zu einem Wert von 20 %.

Entsprechend dem erfindungsgemäßen Verfahren wird der in dem ersten Messdurchgang erhaltene Messwert bis zu einem oberen Grenzwert im Bereich von 5 % bis 6 % übernommen. Anschließend wird in einem zweiten Messdurchgang eine zweite Dosis desselben Gasgemischs 1 mittels desselben Trägergases 3 durch die Trenneinrichtung 4 geleitet, wobei aber im Unterschied zu dem ersten Messdurchgang die Dosis verringert, das Gasgemisch 1 volumetrisch verdünnt und/oder die Trägergasgeschwindigkeit verringert ist. Die beiden erstgenannten Maßnahmen führen unmittelbar, die dritte Maßnahme aufgrund Peakverbreiterung mittelbar zu einer Verringerung der Peakhöhe der abgetrennten Wasserstoff-Fraktion. Da durch die genannten Maßnahmen die Trennleistung des Gaschromatograph herabgesetzt wird, werden mit Ausnahme des Wasserstoffs keine anderen Komponenten des Gasgemischs gemessen.

In Figur 3 zeigt die Kurve C beispielhaft den Zusammenhang zwischen der tatsächlichen Konzentration des Wasserstoffs in dem Gasgemisch und dem in dem zweiten Messdurchgang durch die Wärmeleitfähigkeitsmessung erhaltenen Messwert. Die monotone Abhängigkeit endet hier erst bei einer Konzentration des Wasserstoffs in dem Gasgemisch von etwa 80 %. Wie die Kurve D zeigt, ist jedoch der Messfehler aufgrund des Grundrauschens im Konzentrationsbereich bis etwa 10 % höher als im ersten Messdurchgang. In jedem Fall ist aber der in dem zweiten Messdurchgang erhaltene Messwert geeignet, um den in dem ersten Messdurchgang übernommenen Messwert zu verifizieren. Wenn also der erste Messdurchgang einen Messwert von 5 % und der zweite Messdurchgang einen Messwert von 12 % liefert, beruht der Messwert des ersten Messdurchgangs auf einer Überschreitung des Messbereichs (Grenzwert im Bereich von 5 % bis 6 %), so dass eine entsprechende Fehlermeldung erzeugt wird.

Wie oben bereits erwähnt, wird durch die Verringerung der in den Trägergasstrom 3 injizierten Dosis des Gasgemischs 1, der Verdünnung des Gasgemischs 1, bevor es in die Probenschleife 6 geleitet wird, und/oder durch die Verringerung der Trägergasgeschwindigkeit die Peakhöhe der abgetrennten Wasserstoff-Fraktion, d. h. die Spitzenkonzentration des Wasserstoffs in dem Helium verringert. Indem nun die Dosis, die Verdünnung des Gasgemischs 1 und/oder die Trägergasgeschwindigkeit in dem zweiten Messdurchgang derart eingestellt werden, dass bis zu einer vorgegebenen Konzentration des Wasserstoffs in dem Gasgemisch 1 die Konzentration der an Wärmeleitfähigkeitsdetektor 13 ankommenden abgetrennten Wasserstoff-Fraktion in dem Trägergas 3 kleiner als der oben genannte Grenzwert im Bereich von 5 % bis 6 % ist, wird eine Messbereichserweiterung bis zu dieser vorgegebenen Konzentration, im gezeigten Beispiel etwa 80 %, möglich, wobei allerdings der Messfehler ansteigt. Wird ein Messfehler von etwa 5 % toleriert, so ist eine Messbereichserweiterung in dem zweiten Messdurchgang bis zu einer Konzentration des Wasserstoffs in dem Gasgemisch 1 von etwa 20 % möglich. D. h., Konzentrationen des Wasserstoffs in dem Gasgemisch 1 werden bis zu etwa 5 % bis 6 % in dem ersten Messdurchgang gemessen und in dem zweitem Messdurchgang verifiziert, während darüber hinaus gehende Konzentrationen bis etwa 20 % in dem zweiten Messdurchgang gemessen werden.

Wie Figur 4 zeigt, ist der im ersten Messdurchgang auftretende Messfehler B bis zu einer Konzentration des Wasserstoffs in dem Gasgemisch 1 von etwa 10 % kleiner als der Messfehler aus dem zweiten Messdurchgang. Darüber liefert der zweite Messdurchgang eine höhere Genauigkeit. Es ist daher von Vorteil, bei der Messung von Konzentrationen des Wasserstoffs in dem Gasgemisch 1 oberhalb von etwa 5 % bis 6 % den in dem zweiten Messdurchgang erhaltenen Messwert mit dem Messwert aus dem ersten Messdurchgang zu wichten, indem z. B. ein Mittelwert aus beiden Messwerten bei Wichtung der Messwerte mit dem Kehrwert des geschätzten Messfehlers. Damit erhält man für die Abhängigkeit zwischen dem Messwert und der tatsächlichen Konzentration des Wasserstoffs in dem Gasgemisch 1 den in Figur 4 gezeigten Übergang E von der Kurve A auf die Kurve C und für den resultierenden Messfehler den Übergang F von der Kurve B auf die Kurve D.

## Patentansprüche

1. Verfahren zur chromatographischen Analyse eines Wasserstoff enthaltenden Gasgemischs (1), wobei
- in einem ersten Messdurchgang eine erste Dosis des Gasgemischs (1) mittels Helium als Trägergas (3) durch eine Trenneinrichtung (4) geleitet wird und die Konzentrationen interessierender getrennter Komponenten gemessen werden, wobei mittels eines Wärmeleitfähigkeitsdetektors (13) die Konzentration des Wasserstoffs in dem Gasgemisch (1) gemessen und der dabei erhaltene Messwert bis zu einem oberen Grenzwert im Bereich von 5 % bis 6 % übernommen wird,
- in einem zweiten Messdurchgang eine zweite Dosis desselben Gasgemischs (1) mittels desselben Trägergases (3) durch die Trenneinrichtung (4) geleitet wird, wobei im Unterschied zu dem ersten Messdurchgang die Dosis verringert, das Gasgemisch (1) volumetrisch verdünnt und/oder die Trägergasgeschwindigkeit verringert ist, und erneut mittels des Wärmeleitfähigkeitsdetektors (13) die Konzentration des Wasserstoffs in dem Gasgemisch (1) gemessen wird, und
- der aus dem ersten Messdurchgang übernommene Messwert mit dem in dem zweiten Messdurchgang erhaltenen Messwert verifiziert wird.

2. Verfahren nach Anspruch 1, **dadurch gekennzeichnet, dass** die Dosis, die Verdünnung des Gasgemischs (1) und/oder die Trägergasgeschwindigkeit in dem zweiten Messdurchgang derart eingestellt werden, dass bis zu einer vorgegebenen Konzentration des Wasserstoffs in dem Gasgemisch (1) die Konzentration der an Wärmeleitfähigkeitsdetektor (13) ankommenden abgetrennten Wasserstoff-Fraktion in dem Trägergas (3) kleiner als der Grenzwert im Bereich von 5 % bis 6 % ist, und dass der in dem zweiten Messdurchgang erhaltene Messwert übernommen wird, wenn er unterhalb der vorgegebenen Konzentration liegt und der in dem ersten Messdurchgang erhaltene Messwert wegen Überschreitens des oberen Grenzwerts nicht übernommen worden ist.

3. Verfahren nach Anspruch 2, **dadurch gekennzeichnet, dass** der aus dem zweiten Messdurchgang übernommene Messwert in einem sich an den oberen Grenzwert anschließenden Intervall durch Wichtung mit dem in dem ersten Messdurchgang erhaltenen Messwert korrigiert wird.

## Claims

1. Method for the chromatographic analysis of a hydrogencontaining gas mixture (1), wherein
- in a first measurement procedure, a first dose of the gas mixture (1) is conducted by means of helium as a carrier gas (3) through a separating device (4) and the concentrations of separated components of interest are measured, wherein the concentration of the hydrogen in the gas mixture (1) is measured by means of a thermal conductivity detector (13) and the measured value obtained thereby is accepted up to an upper limiting value in the range from 5% to 6%,
- in a second measurement procedure, a second dose of the same gas mixture (1) is conducted by means of the same carrier gas (3) through the separating device (4), wherein, in contrast to the first measurement procedure, the dose is reduced, the gas mixture (1) is volumetrically diluted, and/or the carrier gas speed is reduced, and the concentration of the hydrogen in the gas mixture (1) is measured again by means of the thermal conductivity detector (13), and
- the measured value accepted from the first measurement procedure is verified using the measured value obtained in the second measurement procedure.

2. Method according to Claim 1, **characterized in that** the dose, the dilution of the gas mixture (1), and/or the carrier gas speed are set in the second measurement procedure in such a manner that up to a predefined concentration of the hydrogen in the gas mixture (1), the concentration of the separated hydrogen fraction, which arrives at the thermal conductivity detector (13), in the carrier gas (3) is less than the limiting value in the range from 5% to 6%, and **in that** the measured value obtained in the second measurement procedure is accepted if it lies below the predefined concentration and the measured value obtained in the first measurement procedure has not been accepted because of exceeding the upper limiting value.

3. Method according to Claim 2, **characterized in that** the measured value accepted from the second measurement procedure is corrected, in an interval following the upper limiting value, by weighting with the measured value obtained in the first measurement procedure.

## Revendications

1. Procédé d'analyse chromatographique d'un mélange (1) gazeux comprenant de l'hydrogène, dans lequel
- dans une première passe de mesure, on envoie une première dose du mélange (1) gazeux, au moyen d'hélium comme gaz (3) porteur, dans un dispositif (4) de séparation et on mesure les concentrations des constituants séparés auxquels on s'intéresse, dans lequel, au moyen d'un détecteur (13) de conductibilité thermique, on mesure la concentration de l'hydrogène dans le mélange (1) gazeux et on prend la valeur de mesure ainsi obtenue jusqu'à une valeur limite supérieure dans la plage de 5 % à 6 %,
- dans une deuxième passe de mesure, on envoie une deuxième dose du même mélange (1) gazeux au moyen du même gaz (3) porteur dans le dispositif (4) de séparation, dans lequel, à la différence de la première passe de mesure, la dose est diminuée, le mélange (1) gazeux est dilué volumétriquement et/ou la vitesse du gaz porteur est diminuée, et on mesure à nouveau, au moyen du détecteur (13) de conductibilité thermique, la concentration de l'hydrogène dans le mélange (1) gazeux, et
- on vérifie la valeur de mesure prise dans la première passe de mesure par la valeur de mesure obtenue dans la deuxième passe de mesure.

2. Procédé suivant la revendication 1, **caractérisé en ce qu'**on règle la dose, la dilution du mélange (1) gazeux et/ou la vitesse du gaz porteur dans la deuxième passe de mesure, de manière à ce que, jusqu'à une concentration donnée à l'avance de l'hydrogène dans le mélange (1) gazeux, la concentration de la fraction d'hydrogène, séparée et arrivant au détecteur (13) de conductibilité thermique, dans le gaz (3) porteur soit plus petite que la valeur limite dans la plage de 5 % à 6 %, et **en ce qu'**on prend la valeur de mesure obtenue dans la deuxième passe de mesure, si elle est en dessous de la concentration donnée à l'avance et si la valeur de mesure obtenue dans la première passe de mesure n'a pas été prise en raison du dépassement de la valeur limite supérieure.

3. Procédé suivant la revendication 2, **caractérisé en ce qu'**on corrige la valeur de mesure prise dans la deuxième passe de mesure dans un intervalle se raccordant à la valeur limite supérieure par pondération par la valeur de mesure obtenue dans la première passe de mesure.
